# EUROPEAN PATENT APPLICATION

(11) **EP 1 892 324 A1**
(43) Date of publication of application: **27.02.2008**
(21) Application number: 06119484.1
(22) Date of filing: 24.08.2006
(51) Int. Cl.: D04H 13/00

(54) **Sheet material having skin protection properties, and use of such material for skin protection**

(71) Applicant: Bio-Racer, 3980 Tessenderlo (BE)
(72) Inventor: Vanstraelen, Raymond, 3780 Tessenderlo (BE); Van Osnabrugge, Joost, 3780 Tessenderlo (BE); De Raeve, Alexandra, 9000 Gent (BE); Godefroidt, Frank, 9000 Gent (BE); Van Parys, Marc, 9000 Gent (BE)
(74) Representative: Leherte, Georges M.L.M.

(57) **Abstract**

The invention relates to a sheet material comprising an elastic fabric layer and a coating layer of a tacky silicone gel, in which the elastic fabric consists of an eyelet type fabric comprising at least 15 % of elastane yarns and the tacky silicone gel coating layer has apertures corresponding to the eyelet-apertures of the elastic fabric, preferably comprising 50 to 1000 eyelets per dm², with an eyelet opening area of at least 5 % of the sheet area ;
to the use of such sheet material for its application to a body part in view of reducing skin friction and/or skin injury during physical exercise and/or sports practice, in particular to the buttocks as protection during bicycle practice ;
to a method for reducing skin friction and/or skin injury during physical exercise ; and
to protective sports accessories or garments for reducing skin friction during sports practice, comprising a piece of such sheet material.

## Description

The invention relates to a method to reduce the consequences of skin friction during physical exercise. Skin frictions resulting from repeated movements, such as occur in work conditions or during sports exercise, like in walking or bicycling, often lead to skin injury.

Numerous methods have been considered and/or developed to avoid, alleviate or cure such injuries. These methods may involve a reduction of the pressure exerted by the objects causing a friction, lubricating means for reducing the friction itself, means to heal the injury once it has occurred, etc., etc... All these approaches provide some benefit in specific situations, but also involve specific drawbacks, either inherently or in specific situations.

There remains therefore clearly a need for alternative methods which may be more satisfying for certain individuals, or provide better results in specific situations.
Such a specific situation is for instance the buttocks area / saddle area of sports cyclists. All "solutions" currently proposed to solve the "saddle sour" problem have shown insufficiently satisfactory in the long run.

It is the objective of the present invention to propose an improved solution to the problems encountered with state of the art materials for protecting the skin during physical exercise, such as in particular the "saddle sour" problem. The approach according to the invention involves the use of a sheet comprising an elastic (bi-elastic) fabric and a coating layer of a tacky silicone gel (cq. gel elastomer).

Such sheets are already known as such, as therapeutic elastic composite materials for treatment and/or rehabilitation of scar tissue, for instance from US patents 4.838.253, 5.340.363 and 5.891.076, and from patent publication WO 02/45698.

According to US 4.838.253 the material may involve a dressing of tacky silicone gel on one surface and a non tacky silicone elastomer on the other surface. The material is reported to be useful in providing cushioning and protection from abrasions and blisters and for use by athletes, in applications in which the user's clothing is likely to come into contact with the outer surface of the material. The presence of silicone material on both surfaces appears however to make it insufficiently effective for the applications contemplated by the present invention, such as avoiding "saddle sour" problems.

US 5.340.363 discloses a liquid permeable sheet material comprising one or more sheets of apertured material coated with a sufficient amount of tacky silicone gel to effectively encapsulate the sheet(s) but insufficient to occlude the apertures. Also this material appears to be inadequate for achieving the objectives of the present invention.

US 5.891.076 relates to a scar dressing material comprising a flexible carrier sheet embedded within a silicone gel such that the gel forms continuous layers on both sides. The pores and cavities of the carrier material are filled with silicone-gel. Due to its tackiness on both surfaces and its rather poor breathability, the material appears to be inappropriate for achieving the objectives of the present invention.

WO 02/45698 discloses a silicone gel sheet comprising an elastic fabric carrying, on the surface contacting the skin, a tacky silicone gel elastomer that does not permeate the fabric, being thus uncoated on the exterior surface. The presence of a non tacky exterior surface is said to be beneficial to avoid the tendency of clothing adhering thereto and to avoid the problem of soiling. A specific embodiment of the sheets can be used on parts of the body for preventing bedsores. The material appears to be inappropriate for achieving the objectives of the present invention.

In European patent application 05106249.5 which was not yet published at the date of filing the present patent application, the applicants proposed the use of a sheet with a discontinuous silicone layer in order to provide "breathing" properties to the sheet. According to European patent application 05106245.9 such a discontinuous breathing layer may consist of discrete dots of silicone gel material, discrete stripes of silicone gel material, continuous stripes of silicone gel material in one or in several directions (for instance in longitudinal and transverse direction).

The present invention now proposes to achieve the objective of an improved sheet material for protecting the skin during physical exercise, by providing a sheet material comprising an elastic fabric layer and a coating layer of a tacky silicone gel (cq. tacky silicone gel elastomer), in which the elastic fabric consists of an eyelet type fabric comprising at least 15 % of elastane yarns and the tacky silicone gel coating layer has (involves, comprises, shows) apertures corresponding to the eyelet-apertures of the elastic fabric.

The expression "eyelet type fabric" as used in this text broadly refers to a fabric comprising an important proportion of "eyelets" or apertures, obtained
either during the fabrication of the fabric itself (by "weaving in" or "knitting in" the apertures (eyelets), or any other technique for "manufacturing in" the apertures into the fabric),
or during a perforation / puncturing step subsequent to the fabric manufacturing, before or during the step of providing the silicone coating layer to the fabric.

According to a further, preferred feature of the invention, the elastic fabric may more in particular comprise from 50 to 1000 eyelets per dm², with an eyelet opening area of at least 5 % of the sheet fabric area, most preferably from 100 to 500 eyelets per dm², with an eyelet opening area of at least 5 - 20 % of the sheet fabric area.

According to still another preferred feature of the invention, the sheet material has a high "breathability".

The expression "breathability" of the coated eyelet fabric according to the invention refers to a low permeating resistance parameter "R" as measured, for instance, by means of a KES-F8 Air Permeability Tester as marketed by KATO TECH CO. LTD.

Typically, preferred sheet materials according to the invention have a permeating resistance parameter "R" in the range below 1 KPa.s/m, preferably as low as 0,01 KPa.s/m.

The elastic fabric used in accordance with the invention preferably comprises at least 20 % of elastane yarns, and, comprises mainly polyester and/or polyamide yarns beside the elastane yarns.

The elastic fabric may most suitably consist of a bi-elastic "lycra"-type fabric. The expression "lycra"-type fabric refers to elastic fabrics comprising a proportion of elastic polyurethane ("spandex", "lycra", "elastane") fibres, as commonly know in the art.
The essence in this context is the bi-elastic behaviour (same or different elasticity in longitudinal and in transverse directions) of the fabric so that the piece of sheet can optimally follow the body part to which it is applied / adhered (with elasticity up to 150% and more).
The fabric is preferably rather thin, for instance a fabric weighing around 210 g/m² (suitable fabrics are those often used nowadays for competition swimsuits and sport cyclist shorts).

According to a preferred feature of the invention the elastic fabric may very suitably consist of an eyelet type knitted fabric.
Suitable fabrics are for instance produced by the company ESCHLER in Switzerland, the company PIAVE MAITEX Spa in Italy, and the company LIEBAERT NV in Belgium.

A most convenient elastic fabric consists, for instance, of a warp knitted eyelet fabric, as produced on a jersey type knitting machine with two eyelet beams ("tricotmachine met 2 ogenbalken" in Dutch, such as a "kettingstoel machine"), using "net-laying" ("Filet-Legung" in German).
More generally the elastic fabric very appropriately may consist of a warp knitted eyelet fabric.

The material can be evenly coloured, or with a coloured pattern or with any decorative or advertising or communicative printing (where printing has to be understood very broadly, including special techniques such as sublimation printing).

According to a further feature of the invention the silicone gel elastomer coating results from a two component silicone system for skin adhesion, formulated in such proportions and cured under such conditions to provide the properties appropriate to the application.

Such preferred silicone systems are commonly known in the art (reference is made in this respect to US 5.891.076 mentioned here above) and commercially available for various applications involving adhesion directly on the skin (such as in particular for breast prosthesis application and for the treatment of burns/fire injuries, etc.), and referred to for instance in patent documents GB-A 2 192 142, EP-A 0 399 520, EP-A 0 251 810 and US 5,919,476.

The essence in the context of the present invention is optimal skin friendliness.

Suitable silicone systems are for instance available from WACKER Silicones (in particular under the reference "ELASTOSYL ® P 7010") and from DOW CORNING (in particular under reference "DOW CORNING® 3631 ").

The proportions of the two components of the silicone system will depend on the required tackiness and flexibility of the coating, in relation with the applied curing times.

The tacky silicone gel elastomer coating on the elastic fabric has most suitably a thickness between 50 µ and 1000 µ, preferably between 50 µ and 300 p, corresponding a preferred coating layer of approximately 50 - 300 g/m² of silicone coating on the elastic fabric.
The silicone gel layer may be applied by any method, as in itself well known in the art, for applying liquid curing systems, like for instance by spatula spreading techniques, transfer techniques, etc.

The invention also relates to the use of the sheet material as defined above, in which said sheet material is applied with the tacky silicone coating side to a body part in view of reducing skin friction and/or skin injury during physical exercise and/or sports practice. Such use may include also medical applications where skin friction / skin injury reduction may be involved.

According to a preferred feature of such use, a piece of such sheet material is adhered to the buttocks as protection against skin friction and/or skin injury during bicycle practice.

The elastic property of the fabric used for the sheet materials according to the invention constitutes a further particularly interesting advantage in the specific use of said sheet materials in sports and/or medical applications, in view of the muscle compression which the sheet material can thus provide.

The invention also relates to a method for reducing skin friction and/or skin injury during physical exercise and/or medical treatment, by applying a protective sheet to a body part to be protected, in which a sheet material according to the invention is applied with the tacky silicone coating side to a body part.

The invention also relates to protective sports accessories or garments for reducing skin friction during sports practice, in which such accessory or garment comprises a piece of sheet material according to the invention, provided to be applied with the tacky silicone coating side to a body parting view of reducing skin friction and/or skin injury during sports practice.

In a preferred embodiment of the invention, such protective sports accessory or garment constitutes a buttocks protection accessory or garment for cycling practice, comprising a piece of sheet according to the invention, provided to be applied with its tacky silicone coating side to the buttocks of a cyclist.

In another preferred embodiment of the invention, the protective accessory or garment constitutes an insert sole for application against the bare foot sole (for instance before putting on a sock or a shoe).

The invention has been disclosed here above in respect of its essential features as required for a skilled art person to be able to put the invention to practice.

Many variants to the invention will be readily apparent to the skilled person, beyond the specific features and details set forth in this disclosure, without departing from the basic concept of the invention.

It is thus clear, for instance, that in garments according to the invention only part of the fabric sheet may be coated with silicone gel elastomer, in those areas where adhesion to the body skin (in particular the buttocks) is required.

It is clear also that for certain specific applications other fabrics than those specifically referred to may be appropriate, such as special "cushioning" fabrics not only reducing friction, but also pressure and/or shocks.
The following (non limiting) example of a specific elastic fabric for use in the present invention is disclosed to illustrate the kind of breathing fabric required for achieving the objectives of the invention.

### Example of a warp knitted eyelet fabric

Elastic fabric consisting of a warp knitted eyelet fabric, produced on a jersey type knitting machine with two eyelet beams ("tricotmachine met 2 ogenbalken" in Dutch), using "net-laying" ("Filet-Legung" in German).

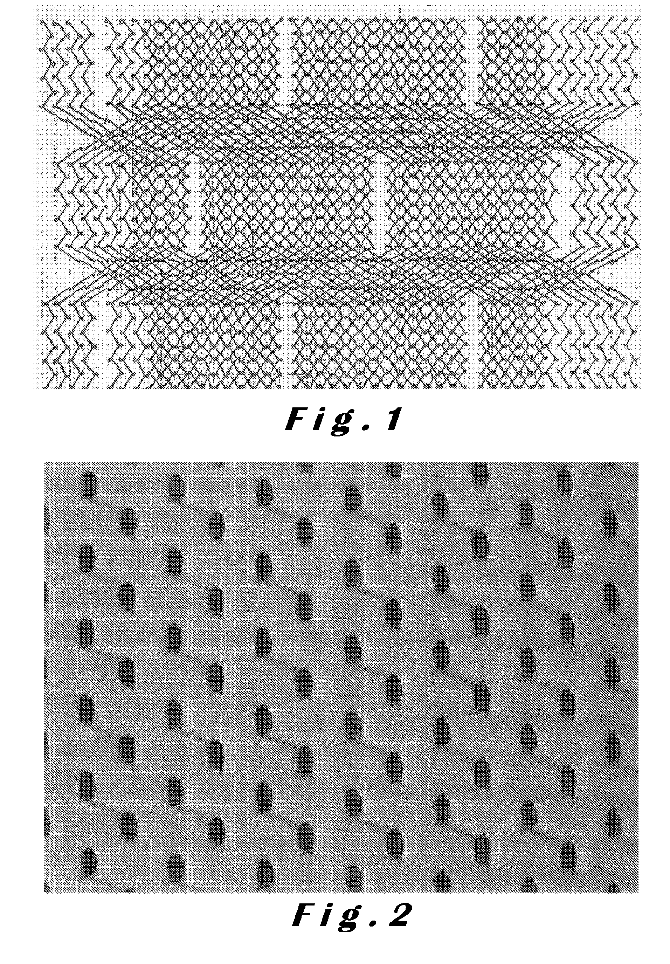

The applied pattern ratio was 20 rows, with on
eyelet beam 1 : 6 rows closed stitch jersey ("gesloten steek tricot" in Dutch) + 4 rows atlas open stitch to the right + 6 rows closed stitch jersey + 4 rows atlas open stitch to the left, and on
eyelet beam 2 : 6 rows closed stitch jersey + 4 rows atlas open stitch to the left + 6 rows closed stitch jersey + 4 rows atlas open stitch to the right ;
so, in other words the two eyelet beams produce the same pattern but with a shift of 10 rows :
applied "pull trough" : 13 full and 1 empty ;
the knitting pattern is represented in figure 1, whereas figure 2 shows a photograph of the obtained fabric.
The elastic warp knitted eyelet knitted fabric shows approximately 300 - 350 eyelets / dm² (in fact ~ 325 eyelets / dm²), with an eyelet opening area of approximately 10 % (in fact ~ 9,75 %) of the sheet fabric area.

## Claims

1. Sheet material comprising an elastic fabric layer and a coating layer of a tacky silicone gel, **characterised in that** the elastic fabric consists of an eyelet type fabric comprising at least 15 % of elastane yarns and the tacky silicone gel coating layer has apertures corresponding to the eyelet-apertures of the elastic fabric.

2. Sheet material according to claim 1, **characterised in that** the elastic fabric comprises from 50 to 1000 eyelets per dm², with an eyelet opening area of at least 5 % of the sheet fabric area.

3. Sheet material according to claim 1, **characterised in that** the elastic fabric comprises from 100 to 500 eyelets per dm², with an eyelet opening area of at least 5 - 20 % of the sheet fabric area.

4. Sheet material according to any one of the preceding claims, **characterised in that** the elastic fabric comprises at least 20 % of elastane yarns.

5. Sheet material according to any one of the preceding claims, **characterised in that** the elastic fabric comprises mainly polyester and/or polyamide yarns beside the elastane yarns.

6. Sheet material according to any one of the preceding claims, **characterised in that** said elastic fabric is a bi-elastic "lycra"-type fabric.

7. Sheet material according to any one of the preceding claims, **characterised in that** the elastic fabric consists of an eyelet type knitted fabric.

8. Sheet material according to claim 7, **characterised in that** the elastic fabric consists of a warp knitted eyelet fabric, as produced on a jersey type knitting machine with two eyelet beams ("tricotmachine met 2 ogenbalken" in Dutch), using "net-laying" ("Filet-Legung" in German).

9. Sheet material according to any one of the preceding claims, **characterised in that** said silicone gel coating results from a two component silicone system for skin adhesion, formulated in such proportions and cured under such conditions to provide the properties appropriate to the application.

10. Use of a sheet material according to any one of claims 1 to 9, **characterised in that** said sheet material is applied with the tacky silicone coating side to a body part in view of reducing skin friction and/or skin injury during physical exercise and/or sports practice.

11. Use according to claim 10, **characterised in that** a piece of such sheet material is adhered to the buttocks as protection against skin friction and/or skin injury during bicycle practice.

12. Method for reducing skin friction and/or skin injury during physical exercise by applying a protective sheet to a body part to be protected, **characterised in that** a sheet material according to claim any one of claims 1 - 9, is applied with the tacky silicone coating side to a body part.

13. Protective sports accessory or garment for reducing skin friction during sports practice, **characterised in that** said accessory or garment comprises a piece of sheet material according to any one of claims 9, provided to be applied with the tacky silicone coating side to a body parting view of reducing skin friction and/or skin injury during sports practice.

14. Buttocks protection accessory or garment for cycling practice, **characterised in that** said accessory or garment comprises a piece of sheet according to any one of claims 1 - 9, provided to be applied with its tacky silicone coating side to the buttocks of a cyclist.
